# EUROPÄISCHE PATENTSCHRIFT

(11) **EP 2 209 505 B1**
(45) Veröffentlichungstag und Bekanntmachung des Hinweises auf die Patenterteilung: **24.08.2016**
(21) Anmeldenummer: 08846101.7
(22) Anmeldetag: 29.10.2008
(51) Int. Cl.: A61L 27/54, A61L 27/42

(54) **VERFAHREN ZUR HERSTELLUNG VON MIT WACHSTUMSFAKTOREN BELADENEN PARTIKELN SOWIE DIE SO ERHALTENEN PARTIKEL**
PROCESS FOR PRODUCING PARTICLES LOADED WITH GROWTH FACTORS, AND THE PARTICLES OBTAINED IN THIS WAY
PROCÉDÉ DE PRODUCTION DE PARTICULES CHARGÉES EN FACTEURS DE CROISSANCE, ET PARTICULES AINSI OBTENUES

(30) Priorität: 29.10.2007 DE 102007051914
(43) Veröffentlichungstag der Anmeldung: 28.07.2010
(73) Patentinhaber: Jennissen, Herbert, 50858 Köln (DE)
(72) Erfinder: ZURLINDEN, Kristin, 45721 Haltern Am See (DE); JENNISSEN, Herbert, 50858 Köln (DE)
(74) Vertreter: Nobbe, Matthias
(86) Internationale Anmeldenummer: PCT/EP2008/064677
(87) Internationale Veröffentlichungsnummer: WO 2009/056567

(56) Entgegenhaltungen:
- EP-A- 1 880 739
- WO-A-02/09788
- WO-A-99/26674
- WO-A-03/043673
- WO-A-2004/084965
- WO-A-2007/053850
- WO-A-2008/098976
- K.ZURLINDEN ET AL.: "Chemical Functionalization of a Hydroxyapatite Based Bone Replacement Material for the Immobilization of Proteins" MATERIALWISSENSCHAFT UND WERKSTOFFTECHNIK., Bd. 36, Nr. 12, 2005, Seiten 820-827, XP002533049 VCH VERLAGSGESELLSCHAFT, WEINHEIM.
- DATABASE MEDLINE [Online] US NATIONAL LIBRARY OF MEDICINE (NLM), BETHESDA, MD, US; März 2007 (2007-03), TURHANI DRITAN ET AL: "Exogenous recombinant human BMP-2 has little initial effects on human osteoblastic cells cultured on collagen type I coated/noncoated hydroxyapatite ceramic granules." XP002533065 Database accession no. NLM17307597 & JOURNAL OF ORAL AND MAXILLOFACIAL SURGERY : OFFICIAL JOURNAL OF THE AMERICAN ASSOCIATION OF ORAL AND MAXILLOFACIAL SURGEONS MAR 2007, Bd. 65, Nr. 3, März 2007 (2007-03), Seiten 485-493, ISSN: 0278-2391

## Beschreibung

Die vorliegende Erfindung betrifft ein Verfahren zur Herstellung von mit Wachstumsfaktoren beladenem teilchenförmigem Material (Partikel), die so erhaltenen Partikel und deren Verwendung zur Verbesserung des Einwachsens von Implantatmaterialien in die Knochensubstanz, insbesondere bei metallischen oder keramischen Materialien, die für Implantate wie künstliche Knochen, Gelenke, Zahnimplantate oder auch Kleinstimplantate Verwendung finden.

Die Implantation künstlicher Gelenke oder Knochen hat in den letzten Jahren eine zunehmende Bedeutung z.B. bei der Behandlung von Gelenkdysplasien oderluxationen bzw. bei Erkrankungen, die auf der Abnutzung von Gelenken als Folge von Gelenkfehlstellungen entstehen können, gewonnen. Die Funktion der Implantate und die für ihre Herstellung verwendeten Materialien, die neben Metallen wie Titan oder Metalllegierungen auch Keramik oder Kunststoffmaterialien wie Teflon oder Polylactiden umfassen können, sind stetig verbessert worden, so dass Implantate nach erfolgreichem Heilungsverlauf in 90-95% der Fälle Standzeiten von 10 Jahren aufweisen können.

Ungeachtet dieser Fortschritte und verbesserter operativer Verfahren bleibt eine Implantation immer noch ein schwieriger und belastender Eingriff, insbesondere da sie mit einem langwierigen Einheilungsprozess des Implantates, der oft monatelange Klinik- und Kuraufenthalte einschließlich Rehabilitationsmaßnahmen umfasst, verbunden sind. Neben den Schmerzen stellen dabei für die betroffenen Patienten die Länge der Behandlungsdauer und die Trennung von der vertrauten Umgebung große Belastungen dar. Ferner verursacht der langwierige Heilungsprozess durch die erforderliche intensive Betreuung hohe Personal- und Pflegekosten.

Das Verständnis der Vorgänge auf der molekularen Ebene, die für ein erfolgreiches Einwachsen eines Implantates erforderlich sind, hat sich in den letzten Jahren bedeutend erweitert. Entscheidend für die Gewebeverträglichkeit eines Implantates sind Strukturkompatibilität und Oberflächenkompatibilität. Die Biokompatibilität im engeren Sinne ist alleine von der Oberfläche bedingt. Auf allen Ebenen der Integration spielen Proteine eine maßgebliche Rolle. Wie nachfolgend erläutert, entscheiden sie bereits während der Implantationsoperation durch die Ausbildung einer initialen adsorbierten Proteinschicht über den weiteren Verlauf der Implantateinheilung, da sich auf dieser Schicht später die ersten Zellen ansiedeln.

Bei der molekularen Interaktion zwischen Implantat, das auch als Biomaterial bezeichnet wird, und Gewebe, findet eine Vielzahl von Reaktionen statt, die streng hierarchisch geordnet zu sein scheinen. Als erste biologische Reaktion findet die Adsorption von Proteinen an der Oberfläche des Biomaterials statt. In der dadurch entstandenen Proteinschicht werden anschließend einzelne Proteinmoleküle beispielsweise durch Konformationsänderungen zu Signalstoffen, die auf der Oberfläche präsentiert werden, umgewandelt oder durch katalytische (proteolytische) Reaktionen werden als Signalstoffe wirkende Proteinfragmente freigesetzt.

Ausgelöst durch die Signalstoffe findet in der nächsten Phase die zelluläre Besiedlung statt, die eine Vielzahl von Zellen wie Leukozyten, Makrophagen, Immunozyten, und schließlich auch Gewebezellen (Fibroblasten, Fibrozysten, Osteoblasten, Osteozyten) umfassen kann. In dieser Phase spielen andere Signalstoffe, sogenannte Mediatoren, wie z.B. Cytokine, Chemokine, Morphogene, Gewebshormone und echte Hormone eine entscheidende Rolle. Im Falle einer Biokompatibilität kommt es schließlich zur Integration des Implantates in den Gesamtorganismus, und idealerweise erhält man ein Permanentimplantat.

Im Lichte von Arbeiten, die in den letzten Jahren auf molekularer Ebene der Osteogenese durchgeführt worden sind, haben chemische Signalstoffe, die sogenannten "bone morphogenic Proteins" (BMP-1-BMP-15), die Einfluss auf das Knochenwachstum besitzen, eine zunehmende Bedeutung gewonnen. BMPs (insbesondere BMP-2 und BMP-4, BMP-5, BMP-6, BMP-7) sind osteoinduktive Proteine, die Knochenneubildung und Knochenheilung stimulieren, indem sie die Proliferation und Differenzierung von Vorläuferzellen zu Osteoblasten bewirken. Darüber hinaus fördern sie die Bildung von alkalischer Phosphatase, Hormonrezeptoren, knochenspezifischer Substanzen wie Kollagen Typ 1, Osteocalcin, Osteopontin und schließlich die Mineralisation.

Die BMP-Moleküle regulieren dabei die drei Schlüsselreaktionen Chemotaxis, Mitose und Differenzierung der jeweiligen Vorläuferzelle. Darüber hinaus spielen BMPs eine wichtige Rolle in der Embryogenese, Organogenese des Knochens und anderer Gewebe, wobei als Zielzellen Osteoblasten, Chondroblasten, Myoblasten und vaskulare glatte Muskelzellen (Proliferationshemmung durch BMP-2) bekannt sind.

Inzwischen sind 15 BMPs inklusive multipler Isoformen bekannt. Bis auf das BMP-1 gehören die BMPs der "transforming growth factor beta" (TGF-ß)- Superfamilie an, für die spezifische Rezeptoren auf den Oberflächen der entsprechenden Zellen nachgewiesen wurden. Wie der erfolgreiche Einsatz von rekombinanten humanen BMP-2 und/oder BMP-7 in Versuchen zu Defektheilungsprozessen an Ratten, Hunden, Kaninchen und Affen gezeigt hat, scheint keine Speziesspezifität vorzuliegen.

Im Stand der Technik ist eine Reihe von Arbeiten auf dem Gebiet beladener Materialien und Partikel, die zur Förderung des Wachsens der Knochensubstanz verwendet werden, bekannt. Berichte über die Bindungen von BMP-2 an Hydroxylapatit (HAP) gehen auf die Anfänge der BMP-Forschung zurück, als von Urist 1984 festgestellt wurde, dass BMP chromatographisch auf einer Hydroxylapatitsäule gereinigt werden kann. Noch im selben Jahr beschrieb Urist ein Aggregat aus BMP und TCP, welches in Mäusen eine Knorpelbildung induzierte (US 4,596,574). In den folgenden 20 Jahren erschien eine Vielzahl von Arbeiten zum Einsatz einer Kombination von Calciumphosphaten (Hydroxylapatit, Tricalciumphosphat) mit BMP-2. Dabei wurde unter anderem beschrieben, dass BMP-2 in einer definierten Menge mit Kollagen oder Hydroxylapatit (HAP) vermengt wird und das Gemisch unmittelbar lyophylisiert und nach Lyophilisierung verwendet wird. In einer anderen Arbeit wird die Adsorption von denaturiertem rhBMP-2 in Gegenwart des Denaturierungsmittels wie Harnstoff an Hydroxylapatit studiert. Selbst unter diesen drastischen Bedingungen werden jedoch nur sehr geringe Mengen BMP-2 an Hydroxylapatit gebunden.

Im Stand der Technik (K. Zurlinden et al., Mat.-wiss. u. Werkstofftech. 2005, 36, No. 12) wird zudem gelehrt, dass Ubiquitin und rhBPM-2 auf Hydroxylapaptit nach dessen chemischen Aktivierung immobilisiert werden können.

Derzeit wird BMP-2 therapeutisch entweder als Induct Os® (Wyeth) auf einem "absorbable collagen sponge" oder in Form des Ossigraft® (Stryker) appliziert. Diesen Materialien ist jedoch gemein, dass die eingesetzte Konzentration von BMP pro Volumeneinheit relativ niedrig ist, d.h. der Volumenbedarf bei ∼2 ml Partikel, bzw. "Sponge" für 1 mg BMP-2. Nur unter unphysiologischen Bedingungen wie extremen pH-Werten im alkalischen bzw. sauren Bereich oder in Gegenwart van Detergentien im Neutralbereich sind größere Mengen an BMP-2 löslich. Diese Mengen sind für eine der Wundgröße angemessenen Applikation des BMP-2 und für eine optimale Stimulation des Knochenwachstums insbesondere in Gegenwart zusätzlicher Knochenersatzstoffe in vielen Fällen nicht ausreichend. Beeinträchtigend kommt hinzu, dass das BMP-2 bei diesen Applikationsformen wegen der nicht ausreichenden Bindung an Kollagen dem Organismus gleichzeitig in einer einzigen frühen Freisetzungsphase ("Burst Phase") zur Verfügung gestellt wird.

Der unten beschriebenen Erfindung liegt die Beobachtung zugrunde, dass durch Adsorption von BMP-2 an teilchenförmigem Material, insbesondere aus anorganischem Knochenersatzmaterial, welches aus Hydroxylapatit, Tricalciumphosphat Calciumcarbonat, Aluminiumoxid oder Mischungen davon besteht, insbesondere bi- oder triphasische Mischungen davon, auf der festen Phase eine höhere Menge an BMP, insbesondere BMP-2 pro Volumenanteil erreicht werden kann als bei den oben erwähnten Materialien, wenn der Adsorptionsschritt über einen ausreichend langen Zeitraum von mindestens 30 Minuten und bei einem kontrollierten pH-Wert zwischen 4 und 5 durchgeführt wird. Nach dem Adsorptionsschritt erfolgt als zweiter Schritt mindestens eine extensive Waschung mit einem gegenüber dem eingesetzten Festphasenvolumen mindestens gleichen Volumen einer Knochenwachstumsfaktor-freien Pufferlösung, bevorzugt mit einem mindestens 10-fachen Flüssigkeitsvolumen. Hierdurch kann sichergestellt werden, dass die Menge an gelöstem BMP-2 in der flüssigen Phase entfernt wird. Dadurch kann eine deutliche Reduktion der sog. Burst-Phase auf 1-2% der adsorbierten BMP-2-Menge erzielt werden. Somit ergibt sich die Möglichkeit der Applikation von BMP-2 in hohen Dosen auf kleinstem Raum. Die Beschichtung der Oberfläche in wässriger gepufferter Lösung erfolgt im sauren Bereich im Bereich zwischen pH 4 und 5, insbesondere bei pH 4,5 erfolgen. Weiterhin ist es von Vorteil, wenn das teilchenförmige Material ein bioresorbierbares Material ist.

Bei dem erfindungsgemäßen Verfahren wird mittels Adsorption als Form der chemischen Bindung, die abzugrenzen ist von:
- Mischung/Kombination mit HAP oder TCP (= Gemenge)
- Einschließen/Entrapping z.B. in Poren
- Inkorporierung/incorporation: z.B. durch Lyophilisierung der Flüssigkeit und Ausfällung im Material
- Beschichtung/Coating von Metallen oder Keramiken, wonach Partikel oder Formkörper z.B. in eine BMP-Lösung eingetaucht und unmittelbar einer Trocknung zur Entfernung der Lösung unterzogen werden [das BMP-2 trocknet also auf der Oberfläche als Schicht ein (= keine Adsorption, sondern Adhäsion)]
unter den Bedingungen des erfindungsgemäßen Verfahrens eine erhöhte Menge an nicht von den Partikeln abwaschbarem Knochenwachstumsfaktor auf der Oberfläche absorbiert. Bei Bindungsstudien von BMP-2 (Tabelle 1) der Erfinder an verschiedenen Hydroxylapatiten wurde überraschend gefunden, dass BMPs, insbesondere BMP-2, über einen weiten Bereich in großen Mengen linear an Calciumphosphate gebunden werden kann.

**Tabelle 1**

| **Adsorption von BMP-2 bei (pH 4.5) und Desorption von BMP-2 (bei pH 7.4) an verschiedenen granulären Knochenersatzmaterialien** | | | | | | | | |
|---|---|---|---|---|---|---|---|---|
| BMP-2 im Inkubationsansatz | Algisorb® (80 %TCP, 20 % HAP) | | Algipore® (98 % HAP) | | Bonit® (13 % SiO₂, 52 % HAP, 35 % TCP) | | NuOss® (HAP, bovin) | |
| | Kontrolle | APS | Kontrolle | APS | Kontrolle | APS | Kontrolle | APS |
| Adsorption von BMP-2, mg/g | | | | | | | | |
| 0,1 | 0,53 | 0,78 | 0,58 | 0,81 | 0,26 | 0,23 | 0,45 | 0,69 |
| 0,2 | 1,06 | 1,54 | 1,27 | 1,52 | 0,52 | 0,39 | 0,61 | 1,16 |
| 0,3 | 1,35 | 2,14 | 1,52 | 2,27 | 0,67 | 0,52 | 0,98 | 1,41 |

| Desorption, t_{1/2}, Tage | | | | | | | | |
|---|---|---|---|---|---|---|---|---|
| Halbwertszeit | 20,4 | 31,6 | | | 10,1 | 5,4 | 28,2 | 30,8 |

In Tabelle 1 sind die Ergebnisse von Adsorptionsversuchen bei pH 4.5 (20 mM Na-Acetat pH 4.5) dargestellt, bei dem die Adsorption/Inkubation über einen Zeitraum von mindestens 30 Minuten (t_{1/2} = 16 d), bevorzugt über mindestens 1-2 Stunden (t_{1/2} = 19 d) und besonders bevorzugt über mindestens 4-6 Stunden (t_{1/2} = 20 d) durchgeführt wird. Längste Halbwertszeiten wurden bei Inkubationen von 15-17 Stunden (t_{1/2} = 23 d) beobachtet.

Nach der Adsorption von BMP-2 auf dem teilchenförmigen Material wird die Freisetzung (Desorption) gemessen. Hierzu werden die Proben in jeweils 2 ml Puffer (50 mM Tris/HCl, 150 mM NaCl, pH 7.4) überführt. Nach festgelegten Zeiten werden die Proben herausgenommen, in 3 x 2 ml Puffer (50 mM Tris/HCl, 150 mM NaCl, pH 7.4) gewaschen und im γ-Counter gemessen. Dann werden sie in 2 ml frischen Puffer für die nächste Periode der Freisetzung überführt. Die Menge an immobilisiertem BMP-2 wird durch die Verwendung von mit ¹²⁵Iod radioaktiv markiertem Protein und Messung im γ-Counter bestimmt.

Alle Knochenersatzmaterialien wurden 15 Stunden bei Zimmertemperatur mit der angegebenen Konzentration BMP-2 in 20 mM Na-Acetat-Puffer pH 4.5 inkubiert. Die Desorption wurde in 50 mM Tris/HCl, 150 mM NaCl, pH 7.4 bestimmt. Die Halbwertszeiten der Freisetzung in der sog. "Burst Phase", die nur 1-2% der adsorbierten BMP-2 Menge betraf, lag zwischen 0.4-1.1 Tagen (nicht gezeigt). APS: Aminopropyltriethoxysilan (nicht unter die Erfindung fallend); Algipore® (Dichte ∼0.63 g/cm³; ®1.3 x 10⁴ Partikel/g) und Algisorb® (Dichte ∼0.63 g/cm³; ∼1.3 x 10⁴ Partikel/g), Fa. Algoss GmbH, Wien; Bonit® Fa. DOT GmbH, Rostock; NuOss® Collagen Matrix ACE Surgical Supply Co. (Brockton, MA, USA).

Die Erfindung wird anhand der beigefügten Figuren weiter erläutert. Dabei zeigen:
Figur 1 die Ultrastruktur eines aus Kalkalgen gewonnenen Algipore^{®} Partikels (Der Maßstabsbalken entspricht 10 µm. Algisorb® besitzt die gleiche Struktur. (Aus "Bone Augmentation in Oral Implantology", Khoury, F. et al., Seite 349, 2007, Quintessenz Verlags-GmbH, Berlin);
Figur 2 den Nachweis der biologischen Aktivität des auf Algisorb adsorbierten rhBMP-2 (C und D) in der Zellkultur mit MC3T3-E1 Zellen mit:
   A. Algisorb, Negativ-Kontrolle - Kein lösliches rhBMP-2 im Medium);
   B. Algisorb, Positiv-Kontrolle - Zusatz von 50 nM löslichem rhBMP-2 zum Medium);
   C. An Algisorb adsorbiertes rh BMP-2 - (∼0.5 mg rhBMP-2 pro g Algisorb)(feucht gehalten);
   D. an Algisorb adsorbiertes rh BMP-2
      (∼0.5 mg rhBMP-2 pro g Algisorb)(getrocknet);

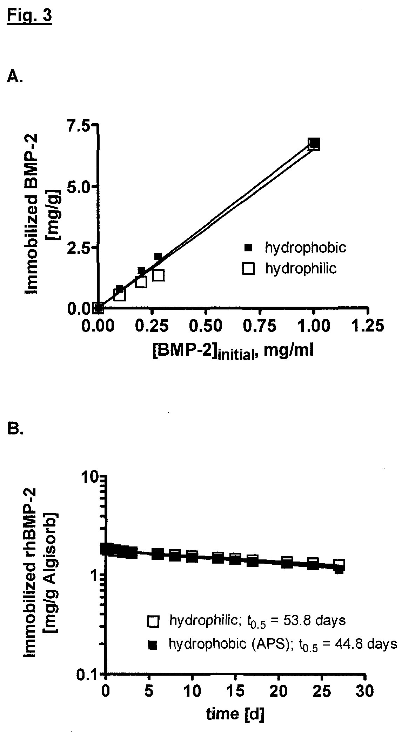

Figur 3A. die hydrophile and hydrophobe Adsorption von rhBMP-2; und
Figur 3B. die Freisetzung von rhBMP-2 (Reaktion 1. Ordnung).

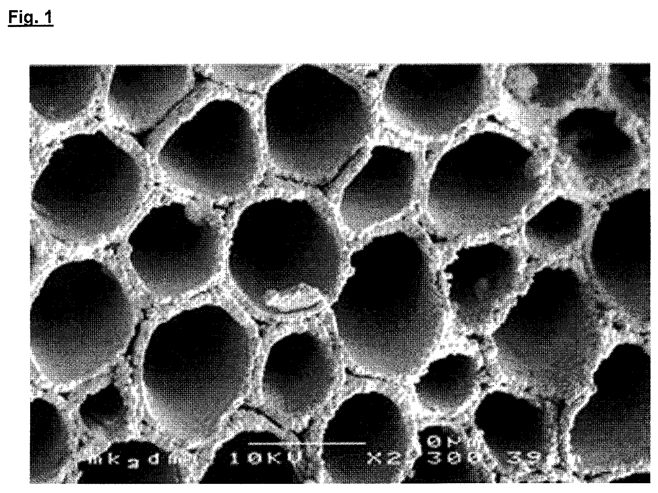

Fig. 1 zeigt eine elektronenmikroskopische Aufnahme des mikrostrukturierten aus einer Kalkalge gewonnenen Algipore® (Fa. AlgOss, Wien), welches ein wesentlich besseres Einheil- und Resorptionsverhalten als andere poröse Hydroxylapatite aufweist. Das ursprüngliche CaCO₃ der roten Kalkalge Cochlearia officinalis wird unter Erhalt der originären Mikrostruktur durch Hydroxylapatit (HAP) (Algipore^{®}) oder Tricalciumphosphat (TCP) (Algisorb^{®}) ersetzt [1].

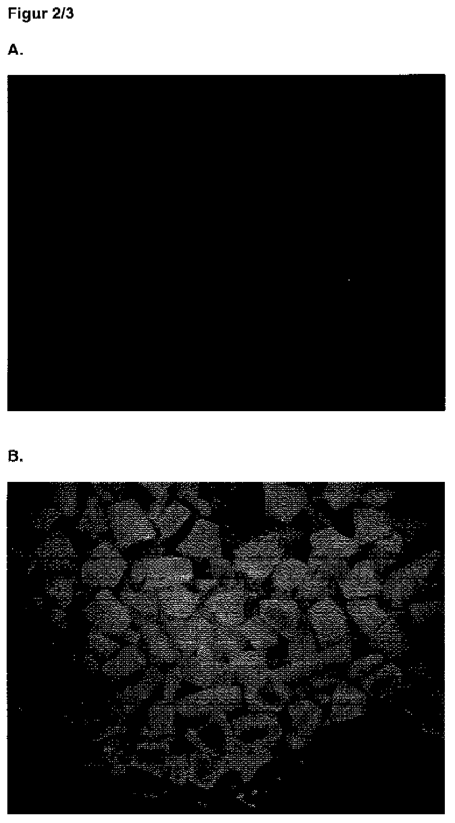

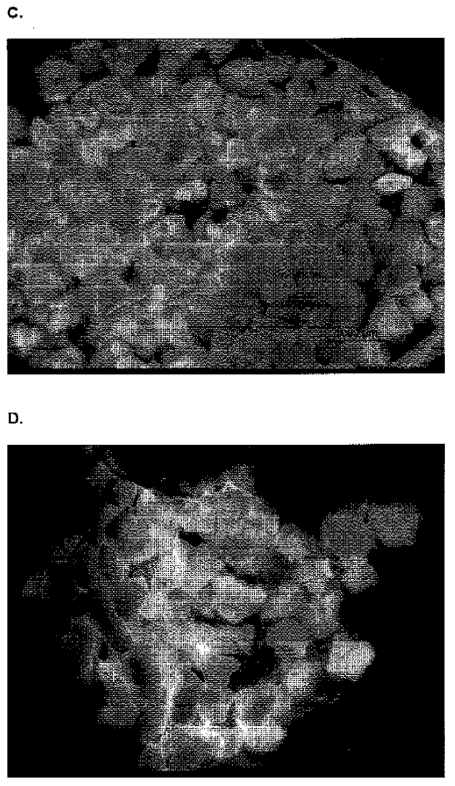

Wie in Figur 2 gezeigt wird, gelang der Nachweis der biologischen Aktivität des auf Algisorb adsorbierten rhBMP-2 in der Zellkultur mit MC3T3-E1 Zellen. Dazu wurden 5x10⁵ frisch trypsinierte MC3T3-E1 Zellen wurden unter sterilen Bedingungen, auf Algisorb-Partikeln, die auf der Unterseite mit Fibrinkleber in den Topfchen einer 48er Mikrotiterplatte befestigt waren, ausgesät und in Alpha-MEM Medium (Gibco) mit 10% FCS inkubiert. 6-12h später wurde das Medium der auf den Plättchen konfluent gewachsenen Zellen durch frisches alpha-MEM Medium mit1% FCS ersetzt und die Zellen wuchsen weiter auf dem Kontroll-Algisorb oder dem Algisorb (ohne Funktionalisierung mit APS) mit adsorbiertem BMP-2 für 6 Tage. Nach 6 Tagen wurden die mit Zellen besiedelten Algisorb-Partikel mit Dulbecco's Phosphatpuffer gewaschen und mit 2% Paraformaldehyd fixiert. Die alkalische Phosphatase (grün fluoreszierender Farbstoff) wurde mit dem Phosphatase-Detektionskit ELF-97 (Molecular Probes,Inc.,Oregon,USA) unter einem Fluoreszenzmikroskop (Nikon Eclipse E400, 10 Megapixel Kamera, Nikon GmbH, Düsseldorf, Germany, Anregungswellenlänge 345 nm, Emissionswellenlänge 530 nm) abgelichtet und bestimmt.

Wie in Figur 3 gezeigt wird, ist hinsichtlich der Eigenschaften des erfindungsgemäßen High-Density Solid-Phase BMP-2 Folgendes ersichtlich. Aus der Partikelzahl von ∼1.3 x 10⁴ Partikel/g Algisorb lässt sich bei einer Beladung mit rhBMP-2 von 6.7 mg/g berechnen, dass 0.5 µg rhBMP-2 pro Partikel gebunden sind. Das bedeutet, dass 2 Partikel (= 1 µg) ausreichen, um im Schafsversuch eine signifikante Knocheninduktion zu erzeugen [2].

Die verbesserten Eigenschaften des Algipore beruhen nach Erkenntnissen der Erfinder zum einen auf dem interkonnektierenden Porensystem und dem Vorliegen von isotropen (d.h. amorphen) Hydroxylapatitpartikeln im Gegensatz zum hochkristallinen Hydroxylapatit in Bio-Oss (Fa. Geistlich). Die Tricalciumphosphat enthaltende Variante der Kalkalge, das Algisorb®, besitzt dabei nach bisherigen Untersuchungen ein noch besseres Resorptionsverhalten als Algipore.

Das Bindungsverhalten wird nicht nur von Algipore und Algisorb gezeigt, sondern bei anderen Hydroxylapatiten findet man ein ähnliches Verhalten. Das Besondere an Algipore und Algisorb ist jedoch, dass die gebundenen Mengen im Bereich von 1-2 mg/g und darüber liegen. Solche Werte sind bisher im Stand der Technik nicht bekannt. Mit dem erfindungsgemäßen Verfahren ist es möglich, über 7 mg BMP2/g Partikel (2.8-4.4 mg/cm³) zu erhalten (Festphasen BMP-2 hoher Dichte, High-density Solid-Phase BMP-2).

Informationen zu den erfindungsgemäß verwendeten Materialien Algipore und Algisorb lassen sich der Referenz [1]entnehmen. Dabei lassen sich erfindungsgemäß besonders Algipore®: 98% Hydroxyapatit HA - monophasisch, und Algisorb®: 80% Tricalciumphosphat ß-TCP, 19.3% HA, 0.7% Calcit CaCO3 - bi/triphasisch einsetzen. Bei letzterem können bei gleicher elektronenmikroskopischer Struktur alle bi/triphasischen Variabeln an ß-TCP und HA eingesetzt werden. Das Calcit beträgt dabei 0.3-0.7%

Untersuchungen der Erfinder zu den Eigenschaften des erfindungsgemäßen High-Density Solid-Phase BMP-2 haben weiterhin ergeben, dass sich aus der Partikelzahl von ∼1.3 x 10⁴ Partikel/g Algisorb bei einer Beladung mit rhBMP-2 von 6.7 mg/g (Fig. 1) berechnen läßt, dass 0.5 µg rhBMP-2 pro Partikel gebunden sind. Das bedeutet, dass 2 Partikel (= 1 µg) ausreichen, um im Schafsversuch eine signifikante Knocheninduktion zu erzeugen [2]. Auf diese Weise lässt sich rhBMP-2 rational und ohne Diffusionsverlust in neuartiger Weise *in vivo* und klinisch applizieren.

Bei der Herstellung des erfindungsgemäßen High-Density Solid-Phase BMP-2 liegt man dann in einem Bereich, bei dem der BMP-2-Gehalt pro Volumeneinheit höher liegt als die Konzentration, die man in wäßrigen Lösungen erhalten kann. Vorzugsweise werden erfindungsgemäß hierzu gepufferte Lösungen von BMP, vorzugsweise BMP-2, mit einer Konzentration von 0,1 bis 1,5 mg/ ml Pufferlösung, vorzugsweise 0,5 bis 1,5 mg/ ml Pufferlösung eingesetzt. Eine so konzentrierte, BMP-enthaltende Pufferlösung wird in einer Menge zu den Partikeln zugegeben, dass sich die gewünschte Beladung in mg BMP pro g Partikel ergibt. Beispielsweise werden 5ml einer 1 mg BMP pro ml enthaltenden Pufferlösung zu 2 g Partikel hinzugegeben, wenn eine Beladung von 2,5 mg BMP pro g Partikel gewünscht ist. Nimmt das Ansatzvolumen im Verhältnis zur Einwaage beispielsweise um das 4fache zu, dann werden aber nur 7 mg/g Partikel statt 10 mg/g Partikel gebunden.

Weitere noch nicht abgeschlossene Untersuchungen der Erfinder zeigen an, dass höhere Werte (von 4-5 mg BMP bis zu 8-10 mg BMP/g Partikel) erhalten werden können. Entsprechend kann eine in der Konzentration darauf abgestimmte Menge in ml an BMP-enthaltender Pufferlösung eingesetzt werden. Es reicht daher bei der Applikation der erfindungsgemäß mit Knochenwachstumsfaktor beladenen Partikel aus, wenn bei einer Applikation nur wenige Körner der BMP-HAP Verbindung eingesetzt werden (z.B. zusammen mit einem Implantat oder bei einer Knochenaugmentation der Kieferhöhle), um eine Knocheninduktion hervorzurufen. *In vitro* Untersuchungen der Erfinder zeigen, dass das an HAP-gebundene BMP-2 biologisch aktiv ist. Weitere Versuche der Erfinder am Schaf laufen derzeit.

Um die Burst-Phase, d.h. die übermässige Freisetzung von Knochenwachtumsfaktor, der nicht an der Oberfläche der Teilchen adsorbiert wurde, sondern lediglich darauf verblieben ist, zu verhindern, werden die Teilchen nach dem Adsorptionsschritt bevorzugt in 3 Waschschritten mit 10fachem Volumen des teilchenförmigem Materials in jeweiliger Knochenwachstumsfaktor-freien Pufferlösung (20 mM Na-Acetat-Puffer, pH 4.5) gewaschen. Danach erfolgt fünfmaliges Waschen in PBS-Puffer pH 7.4 (137 mM NaCl, 8.1 mM Na₂HPO₄, 2.7 mM KCl, 1.5 mM KH₂PO₄, pH 7.4).

Durch Bereitstellung des erfindungsgemäßen High-Density Solid-Phase BMP läßt sich insbesondere rhBMP-2 rational und ohne Diffusionsverlust in neuartiger Weise *in vivo* und klinisch applizieren. Es hat sich gezeigt, dass High-Density Solid-Phase BMP-2 nach Lyophilisierung mehrere Wochen ohne Aktivitätsverlust (nach Fig. 2) lagerfähig ist. Erste Untersuchungen der Erfinder zeigen, dass sich die Lagerfähigkeit des erfindungsgemäßen High-Density Solid-Phase BMP über 1-2 Jahre erstrecken wird. Dabei wird die biologische Aktivität des BMP erhalten, was seitens der Erfinder auch auf die vorzugsweise unter sterilen Bedingungen eingesetzten Materialien zurückzuführen ist.

### Literatur

[1] Spassova, E., Gintenreiter, S., Halwax, E., Moser, D., Schopper, C., & Ewers, R. (2007) Chemistry,Ultrastructure and Porosity of Monophasic and Biphasic Bone Forming Materials Derived from Marine Algae. Materialwiss. Werkstofftech., 38, 1027-1034.
[2] Lichtinger, T.K., Müller, R.T., Schürmann, N., Wiemann, M., Chatzinikoleidou, M., Rumpf, H.M., & Jennissen, H.P. (2001) Osseointegration of Titanium Implants by Addition of Recombinant Bone Morphogenetic Protein 2 (rhBMP-2). Materialwiss. Werkstofftech., 32, 937-941.

## Patentansprüche

1. Verfahren zur Herstellung von mit Wachstumsfaktoren beladenem teilchenförmigem anorganischem Material, bei dem ein teilchenförmiges anorganisches Material, das aus Hydroxylapatit, Tricalciumphosphat, Calciumcarbonat, Aluminiumoxid oder Mischungen davon besteht, mit Wachstumsfaktoren in wässriger gepufferter Lösung im sauren Bereich zwischen pH 4 und 5 über einen Zeitraum von mindestens 30 Minuten behandelt wird, von der wässrigen gepufferten Lösung abgetrennt wird und mindestens einmal mit dem mindestens gleichen Volumen einer Knochenwachstumsfaktor-freien Pufferlösung gewaschen wird.

2. Verfahren nach Anspruch 1, bei dem das teilchenförmige Material nach dem Waschschritt mit der Knochenwachstumsfaktor-freien Pufferlösung getrocknet wird.

3. Verfahren nach Anspruch 2, bei dem der Trocknungsschritt eine Lyophilisierung umfasst.

4. Verfahren nach Anspruch 1, 2 oder 3, bei dem die wässrige gepufferte Lösung einen pH-Wert von 4,3 bis 4,7, insbesondere 4,5, hat.

5. Verfahren nach einem der Ansprüche 1 bis 4, bei dem das teilchenförmige Material eine Teilchengröße im Bereich von 10 bis 500 µm hat und eine interkonnektierende Porenstruktur besitzt.

6. Verfahren nach einem der vorhergehenden Ansprüche, bei dem man als Wachstumsfaktoren BMP-2 oder BMP-7 verwendet.

7. Teilchenförmiges Material, erhältlich nach dem Verfahren nach einem der vorhergehenden Ansprüche.

8. Verwendung des teilchenförmigen Materials nach Anspruch 7 zur Herstellung einer pharmazeutischen Zusammensetzung zur Steigerung des Knochenwachstums.

## Claims

1. Process for preparing a particulate inorganic material loaded with growth factors, wherein a particulate inorganic material, consisting of hydroxyl apatite, tricalcium phosphate, calcium carbonate, aluminum oxide or mixtures thereof, is treated with growth factors in aqueous buffered solution in the acidic range between pH 4 and 5 over a period of at least 30 minutes, the particulate material is separated from the aqueous buffered solution and washed at least once with the same volume of the buffered solution free of bone growth factor.

2. Process according to claim 1, wherein the particulate material is dried after the washing step with the aqueous buffered solution free of bone growth factor.

3. Process according to claim 2, wherein the drying step comprises a lyophilisation.

4. Process according to claims 1, 2 or 3, wherein the aqueous buffered solution has a pH-value of 4,3 to 4,7, in particular 4,5.

5. Process according to any one of claims 1 to 4, wherein the particulate material has a particle size in the range of 10 to 500 µm and an interconnecting pore structure.

6. Process according to any one of the foregoing claims, wherein BMP-2 or BMP-7 is used as growth factor.

7. Particulate material, obtainable according to the process of any one of the foregoing claims.

8. Use of the particulate material of claim 7 for preparing a pharmaceutical composition for increasing bone growth.

## Revendications

1. Procédé de production d'un matériau inorganique particulaire chargé en facteurs de croissance, selon lequel un matériau inorganique particulaire qui est constitué d'hydroxyapatite, de phosphate tricalcique, de carbonate de calcium, d'oxyde d'aluminium ou de mélanges de ceux-ci est traité avec des facteurs de croissance dans une solution aqueuse tamponnée dans le domaine acide compris entre pH 4 et 5 sur une période d'au moins 30 minutes, séparé de la solution aqueuse tamponnée et lavé au moins une fois avec au moins le même volume d'une solution tampon exempte de facteur de croissance osseuse.

2. Procédé selon la revendication 1, selon lequel le matériau particulaire est séché après l'étape de lavage avec la solution tampon exempte de facteur de croissance osseuse.

3. Procédé selon la revendication 2, selon lequel l'étape de séchage comprend une lyophilisation.

4. Procédé selon l'une des revendications 1, 2 ou 3, selon lequel la solution aqueuse tamponnée a un pH de 4,3 à 4,7, en particulier de 4,5.

5. Procédé selon l'une des revendications 1 à 4, selon lequel le matériau particulaire possède une taille de particules comprise dans la plage de 10 à 500 µm et possède une structure de pores interconnectés.

6. Procédé selon l'une des revendications précédentes, selon lequel on utilise de la BMP-2 ou de la BMP-7 comme facteurs de croissance.

7. Matériau particulaire, pouvant être obtenu selon le procédé selon l'une des revendications précédentes.

8. Utilisation du matériau particulaire selon la revendication 7 pour produire une composition pharmaceutique destinée à augmenter la croissance osseuse.
